# EUROPEAN PATENT SPECIFICATION

(11) **EP 1 370 243 B1**
(45) Date of publication and mention of the grant of the patent: **09.07.2008**
(21) Application number: 02701872.0
(22) Date of filing: 06.03.2002
(51) Int. Cl.: A61K 9/14, A61K 31/4439, A61K 9/50, A61P 1/04

(54) **METHOD TO OBTAIN MICROPARTICLES CONTAINING A H+, K+ -ATP-ASE INHIBITOR**
VERFAHREN ZUR GEWINNUNG VON MIKROPARTIKELN MIT A H+, K+ -ATP-ASE-INHIBITOR
PROCEDE DE PREPARATION DE MICROPARTICULES CONTENANT UN INHIBITEUR DE H+, K+ -ATP-ASE

(30) Priority: 09.03.2001 SE 0100822
(43) Date of publication of application: 17.12.2003
(73) Proprietor: AstraZeneca AB, 151 85 Södertälje (SE)
(72) Inventor: GLAD, Hakan, S-431 83 Mölndal (SE); SÖDERBOM, Malin, S-431 83 Mölndal (SE)
(86) International application number: PCT/SE2002/000400
(87) International publication number: WO 2002/072071

(56) References cited:
- WO-A1-99/18935
- DE-A1- 19 733 094
- US-A- 4 946 654
- US-A- 5 628 800
- US-A- 5 817 338
- US-A- 5 883 047
- US-B1- 6 174 548

## Description

### Field of invention

The present invention provides microparticles containing an acid labile H⁺,K⁺-ATPase inhibitor and a method of obtaining such microparticles using a fluid-bed granulation technique.

### Background of the invention

The strategy for the development of a pharmaceutical formulation of a given drug depends on different factors. Ultimately, these factors emanate from 1) the therapeutic needs, 2) the physical and chemical properties of the drug, and 3) the influence from the biological environment where the formulation should release its contents. Thus, both technical and biopharmaceutical considerations will contribute to a successful therapy.

Of special importance to the present invention is formulating an acid labile H⁺,K⁺-ATPase inhibitor with a suitable carrier material in the form of microparticles. Such a formulation contains a multitude of discrete delivery units that can be coated with a suitable pH sensitive, semipermeable or other polymeric film such as an enteric coating. Several advantages can be obtained with this type of formulation compared to conventional tablets. The small size of the microparticles assures a fast and predictable emptying from the stomach and controllable plasma levels of the absorbed drug. From a technological point of view, microparticles are more suitable for coating and handling since a technical fault during the process is fatal for single unit formulations but less so for multiple unit formulations comprising micropellets. Also, microparticle formulations are more versatile for use in different dosage strengths.

An ideal method for the preparation of microparticles where the drug is homogeneously distributed should be simple, reproducible, rapid and independent on the solubility characteristics of the drug. A high product yield of the active substance in the final microparticles should also be obtained.

Several different techniques are available for making microparticles, e.g., fluidized bed spray granulation, spray-drying, extrusion-spheronization, spray-chilling, emulsion solvent evaporation/extraction and coating of nonpareil spheres among others. A review by Conti et al. STP Pharma. Sci. 7, 331 (1997) discusses the technical aspects of coacervation, spray-drying, emulsion solvent extraction, and emulsion solvent evaporation.

However, existing techniques suffer from one or more drawbacks. In extrusion spheronization and in coating of non-pareil particles it has been difficult to achieve acceptable microparticles in the range of 50 - 400 µm or microparticles having a high drug content. Pellets made by these methods contain significant amounts of inert excipients.

In emulsifion solvent evaporation, an emulsion has to be made which restricts the use of the drug. Another drawback is the toxicity of the solvent used, usually methylene chloride, which can remain in the microparticles after drying.

Despite many different approaches there is no disclosed technique that can produce both small microparticles containing a high drug content of acid labile H⁺,K⁺-ATPase inhibitors and microparticles of uniform size. Small microparticles of uniform size improves segregation and dose variation during further processing into capsules or tablets. Further, the existing techniques do not cover several desirable aspects such as the possibility to produce spherical microparticles of different size ranges that are homogeneous, have a high content of an acid labile H⁺,K⁺-ATPase inhibitor and sufficient mechanical strength (to, e.g., withstand coating processes) into one single technique.

There are numerous known processes for preparing granular material using fluidized bed apparatuses. An overview of such processes can be found in, e.g., Aulton (Eds) "Pharmaceutics, The science of dosage form design" Churchill Livingstone, 1988. Basically, fluidization is the operation by which solids are transformed into a fluid like state through the suspension in a gas. When the fluid in a bed entrains large amounts of solid particles, a steady state can be achieved by collecting the entrained particles and returning them to the bed. Such a system is often referred to as a fluid bed. Fluidized beds are often used for granulation or coating of a product. Granulation is typically performed by spraying droplets of a liquid on particles, which are kept in a fluidized state. The liquid which is sprayed wets the surface of the solid particles and then solidifies by drying, or cooling. In this way, particles grow. Coating is usually performed by spraying a solution of coating agents onto the particles.

A process for preparing granules using a fluidized bed process was presented in US Patent No. 4,946,654. Here, however, there is no teaching regarding how to prepare homogeneous microparticles with a high drug load of at least 80% by weight of an acid labile H⁺\K⁺- ATPase inhibitor.

WO 99\59544 describes a method of producing granules using a fluidized bed process. The granules were prepared by using a sugar nucleus and then coating the sugar nucleus with the agent of interest and an enteric coating. The average particle diameter of the granules is between 300-400 µm. The application fails to teach to how to prepare homogeneous microparticles (i.e., microparticles without a sugar nucleus) which contain at least 80% by weight of an acid labile H⁺\K⁺- ATPase inhibitor.

US 6 174 548 discloses granules comprising omeprazole, which have been produced by fluidized granulation. The discloses a content of omeprazole is from 5 to 70 wt % and preferably 10 to 30 wt % but no size distribution of less than 250 µm.

DE 197 33 094 A1 discloses granulation of ascorbic acid into granules comprising 85 to 98 % ascorbic acid in a fluidized bed. The document does not disclose a process for producing micro particles having a size of less than 250 µm, or particles having a mechanical strength enduring coating and compressing.

### Object of the invention

An object of the present invention is to provide a method for preparing a homogeneous microparticle which includes an acid labile H⁺,K⁺- ATPase inhibitor, or an alkaline salt thereof, or one of its single enantiomers, or an alkaline salt thereof. Another object is to provide a method for preparatoion of a microparticle with high amounts of an incorporated H⁺,K⁺- ATPase inhibitor in a high-yield process, e.g., to provide homogeneous microparticles with at least 80% by weight of an H⁺,K⁺- ATPase inhibitor based on the dry content of the microparticle. Also, the invention provides a method to prepare a homogeneous microparticle with an incorporated H⁺,K⁺- ATPase inhibitor that has low friability and sufficient mechanical strength, such that the microparticle can endure coating and compressing processes.

### Disclosure of the invention

It has been found that spherical, free-flowing, homogeneous microparticles containing H⁺,K⁺- ATPase inhibitors having low friability can be obtained by spraying a suspension/solution/emulsion containing an H⁺,K⁺- ATPase inhibitor into a fluidized bed thereby forming granules having a size distribution of between 50 µm to 250 µm, e.g., a size distribution of between 50-200 µm, 50-150 µm or 100-180 µm, and selecting out\separating these microparticles from the fluidized bed. The microparticles produced by the method described herein are nearly spherical in shape, have a smooth surface and have a narrow size distribution. These characteristics ensure that the microparticles can be coated in a predictable and reproducible manner.

More specifically, the method of the present invention includes spraying into droplets a liquid medium having a high dry volume content into a fluidized bed. The liquid medium includes: (i) an acid labile H⁺,K⁺- ATPase inhibitor, or an alkaline salt thereof, or one of its single enantiomers, or an alkaline salt thereof, (ii) a water soluble or non-water soluble polymer, wherein the polymer is at least 5% by weight based on the dry content, and (iii) a liquid in which the polymer is soluble or dispersible. The dry content of the liquid medium can be in the range between 15 to 60 vol %. The solid content may also be expressed as 15-70 weight % (corresponds to 10 to 60 vol %). The content of the H⁺,K⁺- ATPase can be from 80 to 95 weight % of the weight of the dried microparticles. The polymer can be a water soluble or non-water soluble polymer. Preferably, the polymer is a water soluble polymer. The polymer used in the present invention can act as a binder, plastizer and\or a dispersing agent, and can be any polymer known in the art, e.g., a cellulose derivative, e.g., hydroxypropyl methyl cellulose (HPMC), a polysaccharide, a natural polymer, a synthetic polymer, a surfactant and mixtures thereof. The liquid in which the polymer is soluble can be water, tertiary butyl alcohol, cyclohexane, methylene chloride, methanol, ethanol and mixtures thereof.

It was surprisingly found that microparticles of a very small size distribution of less than 250 µm could be produced. These particles have good mechanical strength and can be coated with one or more polymeric film coatings such as an enteric coating. Optionally, a separating layer can be applied before the enteric coating.

Unless otherwise defined, all technical and scientific terms used herein have the same meaning as commonly understood by one of ordinary skill in the art to which this invention belongs. In the case of conflict, the present invention, including definitions will control. All publications, patents, and other references mentioned herein are incorporated by reference.

### H⁺K⁺-ATPase inhibitors

H⁺K⁺-ATPase inhibitors, also named as gastric proton pump inhibitors, are for instance compounds known under the generic names omeprazole, esomeprazole, lansoprazole, pantoprazole, rabeprazole and leminoprazole.

H⁺K⁺-ATPase inhibitors for use in the method described herein include compounds of the general formula I, or an alkaline salt thereof, or one of its single enantiomers, or an alkaline salt thereof. 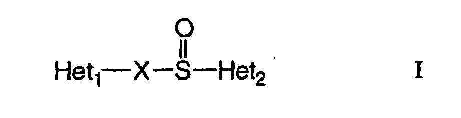 wherein
Het₁ is 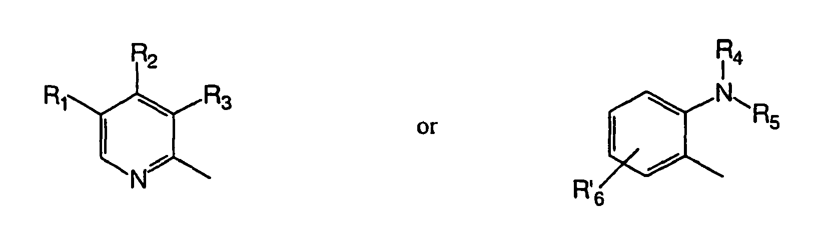 Het₂ is 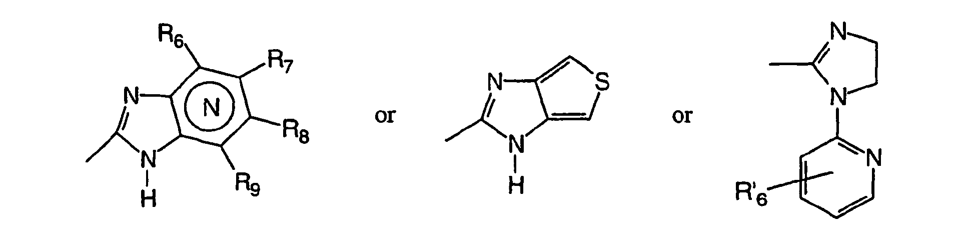 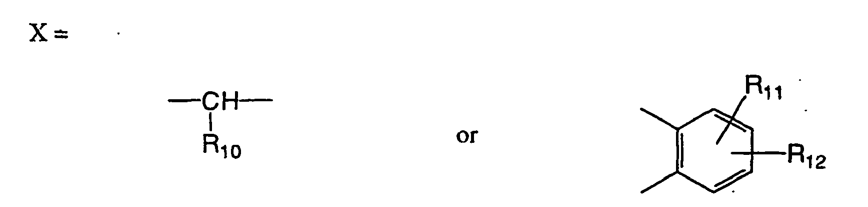 wherein
N in the benzimidazole moiety means that one of the carbon atoms substituted by R₆-R₉ optionally may be exchanged for a nitrogen atom without any substituents;
R₁, R₂ and R₃ are the same or different and selected from hydrogen, alkyl, alkoxy optionally substituted by fluorine, alkylthio, alkoxyalkoxy, dialkylamino, piperidino, morpholino, halogen, phenyl and phenylalkoxy;
R₄ and R₅ are the same or different and selected from hydrogen, alkyl and aralkyl;
R'₆ is hydrogen, halogen, trifluoromethyl, alkyl and alkoxy;
R₆-R₉ are the same or different and selected from hydrogen, alkyl, alkoxy, halogen, haloalkoxy, alkylcarbonyl, alkoxycarbonyl, oxazolyl, trifluoroalkyl, or adjacent groups R₆-R₉ form ring structures which may be further substituted;
R₁₀ is hydrogen or forms an alkylene chain together with R₃ and
R₁₁ and R₁₂ are the same or different and selected from hydrogen, halogen, alkyl or alkoxy.

The alkyl and alkoxy substituents or moieties of substituents are independently a branched or straight C₁-C₉ chain or a cyclic alkyl.

Examples of specifically interesting compounds according to formula I are:

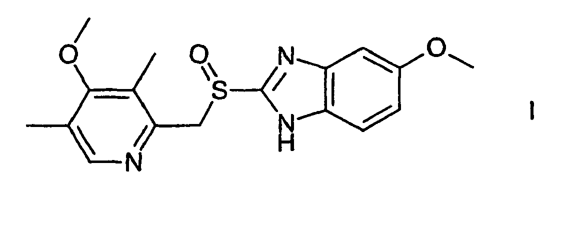

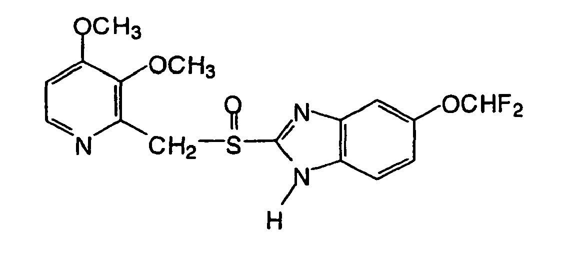

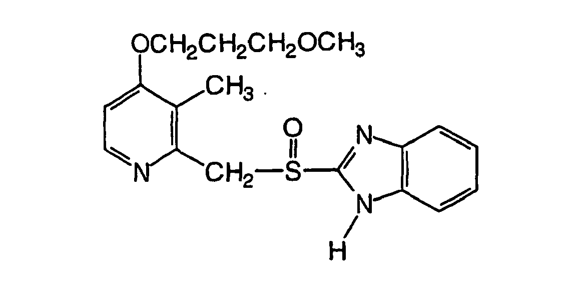

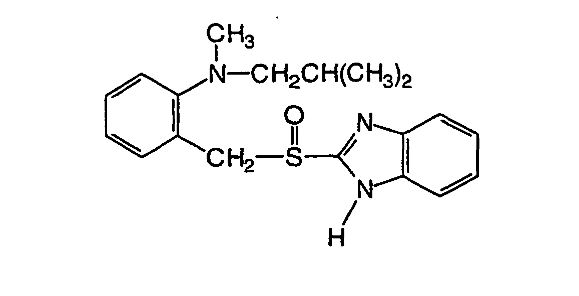

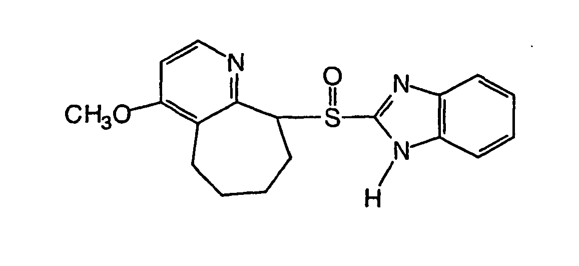

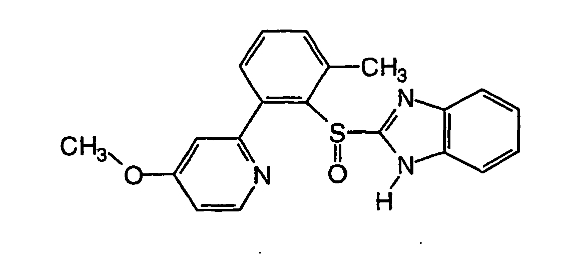

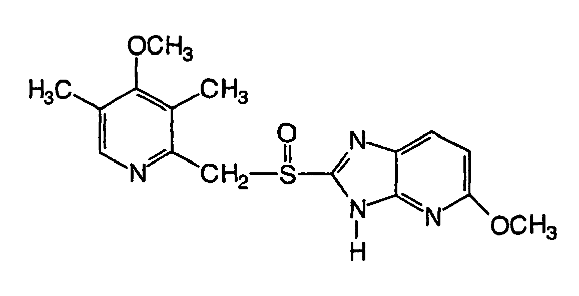

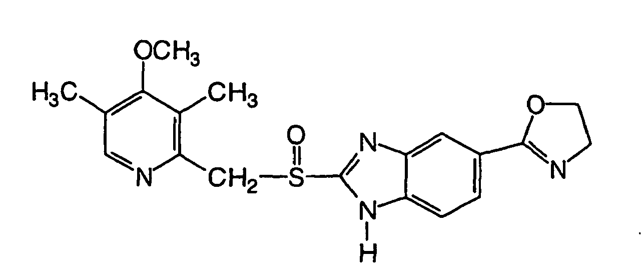

The H⁺,K⁺-ATPase inhibitor used in the method of the invention may be in neutral form, or in the form of an alkaline salt, such as for instance the Mg²⁺, Ca²⁺, Na⁺ or K⁺ salts, preferably the Mg²⁺ salts. Alternatively, one of the single enantiomer or an alkaline salt thereof is used in the method of the invention.

The H⁺,K⁺-ATPase inhibitor used in the invention can be one particular H⁺,K⁺-ATPase inhibitor (e.g., omeprazole, an alkaline salt thereof, esomeprazole or the alkaline salt thereof), a combination of different H⁺,K⁺-ATPase inhibitors, or a combination of an H⁺,K⁺-ATPase inhibitors and another pharmaceutical active ingredient.

Various different types of H⁺,K⁺-ATPase inhibitors are disclosed in EP-A1-0005129, EP-0652872, EP-0124495, EP-0707580, EP-A1-174726, EP-A1-166287 and GB 2163747.

### Polymers

As used herein the term polymer is intended to include any substance that can act as a binder, dispersing agent or plastizer. The polymer can be, but is not limited to an excipient listed below:
- *cellulose derivatives*, like ethylcellulose, hydroxypropyl methyl cellulose, hydroxyethyl cellulose, hydroxypropyl cellulose, ethyl hydroxyethyl cellulose, carboxymethyl cellulose, cellulose acetate butyrate, cellulose acetate phtalate, methylcellulose, etc
- *other polysaccharides*, like alginate; xanthan; carrageenan; scleroglucan; pullulan; dextran; hyaluronic acid; chitin; chitosan; starch; etc
- *other natural polymers*, like proteins (e g albumin, gelatin, etc); natural rubber ; *gum arabic; etc*
- *synthetic polymers*, like acrylates (e g polymethacrylate, poly(hydroxy ethyl methacrylate), poly(methyl methacrylate), poly(hydroxy ethyl methacrylate - co methyl methacrylate), Carbopol® 934, etc); polyamides (e g polyacrylamide, poly(methylen bisacrylamide), etc); polyanhydrides (e g poly(bis carboxyphenoxy)methane, etc); PEO-PPO block-co-polymers (e g poloxamers, etc); polyvinyl chloride; polyvinyl pyrrolidone; polyvinyl acetate; polyvinyl alcohol; polyethylene, polyethylene glycols and co-polymers thereof; polyethylene oxides and co-polymers thereof; polypropylene and co-polymers thereof; polystyrene; polyesters (e g poly(lactid acid), poly(glycolic acid), poly(caprolactone), etc, and co-polymers therof, and poly(ortho esters), and co-polymers thereof); polycarbonate; cellophane; silicones (e g poly (dimethylsiloxane), etc); polyurethanes; synthetic rubbers (e g styren butadiene rubber, isopropene rubber, etc); etc
- *surfactants*, i.e., anionic, like sulphated fatty alcohols (e g sodium dodecyl sulphate), sulphated polyoxyethylated alcohols or sulphated oils, etc; cationic, like one from the group of quaternary ammonium and pyridinium cationic surfactants, etc; non-ionic, like one from the group of polysorbates (e g Tween), sorbitan esters (e g Span), polyoxyethylated linear fatty alcohols (e g Brij), polyoxyethylated castor oil (e g Cremophor), polyoxyethylated stearic acid ( e g Myrj), etc; etc
- *other substances*, like shellacs; waxes (e g carnauba wax, beeswax, glycowax, castor wax, etc); nylon; stearates (e g glycerol palmitostearate, glyceryl monostearate, glyceryl tristearate, stearyl alcohol, etc); lipids (e g glycerides, phospholipids, etc); paraffin; lignosulphonates; mono- or disaccharides (e.g. lactose, etc.); sugar alcohols (e.g. mannitol etc.); etc.

Also, combinations of these excipients are possible.

The excipients mentioned above may be made more ductile by introducing a plasticizer. The plasticizer could be but is not limited to the plasticizers mentioned below:
- glycerol, polyethylene glycol, propylene glycol, triethyl citrate, diethyl phthatate, dibutyl phthalate, dibutyl sebacate, sorbitol, triacetin, etc.

Also, combinations of these plasticizers are possible.

### Low friability microparticles containing acid labile H⁺,K⁺-ATPase inhibitors

Generally the following conditions are used to obtain low friability microparticles according to the method of the invention.

To obtain low friability microparticles the solid content of the suspension/solution/emulsion should be high, and can for instance be in the range of 10 to 70 weight %, 10 - 60 weight %, 15-70 weight % and 20 - 60 weight %. Expressed otherwise, low friability microparticles, that can for instance endure coating with a polymeric film, are achieved when the suspension/solution/emulsion has a solid volume content equal to or higher than 10 vol % and preferably higher than 15 weight %, preferably up to 60 weight %. A microparticle having a high total content of the H⁺,K⁺-ATPase inhibitor can be obtained, for example, as much as 80 weight %, e.g., 85 weight %, 90 weight %, or 95 weight % (based upon the weight of the dried microparticle). The pore size of the obtained microparticles being preferably less than 5.0 µm. Solid content and solid volume content are weight % and volume %, respectively, of dry material in the suspension/solution/emulsion (dry/(dry + liquid)), wherein the dry material is a H⁺,K⁺-ATPase inhibitor + polymer and\or dispersing agent.

According to the present invention homogeneous microparticles can be obtained wherein the solid volume content is from 15 to 60 vol % giving dry compact microparticles. The solid content may also be expressed as 15 to 70 weight % (corresponds to 10 to 60 vol %).

The content of the H⁺,K⁺-ATPase inhibitor calculated on the weight of the dried microparticles are from 80 to 95 weight %, for example from 90 to 95 weight %.

The solid content of the liquid medium is defined as the residue after drying at 110°C for 2 hours, divided by the total amount before drying. The solid content can be expressed either as weight percent or preferably as volume percent.

A microparticle according to the present invention comprises one (or more) H⁺,K⁺-ATPase inhibitors, with one (or more) additional active or non-active substances, which are dispersed within the microsphere.

### Methods of making microparticles

The spherical, free-flowing, homogeneous microparticles described herein can be obtained using any known fluidized bed granulation process, e.g., as described in U.S. Patent No. 4,946,654. A preferred method of forming the homogeneous microparticles includes using a continuous fluid-bed granulation process which has an integrated microparticle selecting system that selects microparticles having a desired size distribution, e.g., microparticles having a size distribution of less than 250 µm. In such a continuous fluid-bed granulation process, there is an external equilibrium between the supply of granulation liquid and the discharge of microparticles and the internal equilibrium between the granulation and nucleation processes. Both equilibrium states are directly related to each other. On the side of granulation liquid supply, the optimal spraying of the granulation liquid creates the condition for granulation and nucleation to take place, on the side of the microparticles selection, deliberate continuous selection ensures that only microparticles of the desired grain size are removed from the process.

The following general steps of the procedure are further exemplified in the experimental section.
*a*) Preparation of a granulation liquid medium for atomizing. The medium is a suspension, a solution or an emulsion of the acid labile H⁺,K⁺-ATPase inhibitor. A suspension is prepared by dissolving or dispersing a polymer in a liquid (as defined below), and then adding fine particles of the acid labile H⁺,K⁺-ATPase inhibitor. A further dispersing agent, e.g., a surfactant, might also be included to facilitate the dispersion of the active substance. The polymer might then act as a binder between the fine active substance particles in the microparticles and can be either a water soluble or a non-water soluble polymer.
*b*) Spraying the acid labile H⁺,K⁺-ATPase inhibitor containing suspension/solution/emulsion is fed through a nozzle, e.g., a pneumatic nozzle, an ultrasonic nozzle, a rotary atomizer or a pressurized nozzle. If two pneumatic nozzles are used, the liquid medium and the air can be alternatively mixed outside the nozzle. The atomization gas used can be any gas which is inert under the operating conditions. Generally, the desired spray droplet diameter is of the order of 10-50 µm.
   In the fluid-bed granulation process, a bottom-up flow of air or inert gas fluidizes the solid acid labile H⁺,K⁺-ATPase inhibitor particles. In the fluidized state, the solid particles are separated from each other and can be efficiently wetted with granulation liquid medium. If a spray droplet hits a particle, the granulation liquid medium spreads over the surface of the particle, ideally forming a complete liquid film. The intensive exchange of heat and matter between the solid particles and the gas stream accelerates drying and aids the solidification of the liquid film on the surface all over the particle. The repeated application and solidification of the liquid spray causes the particle to grow by layers and form a microparticle. The microparticle is compact and also nearly spherical.
   The growth of particles starts in the fluidized bed from nuclei. Thus, for the granulation process to start, the fluidized-bed apparatus can already contain starting granulate, e.g., crystalline particles of the acid labile H⁺,K⁺-ATPase inhibitor. However, it is possible to start granulation in an empty fluidized-bed apparatus. In this embodiment, a spray droplet can be sprayed into an empty fluidized-bed apparatus. Once dry, the droplet can serve as a nucleus.
   Nuclei can be constantly formed in the fluidized bed. For example, spray droplets containing the acid labile H⁺,K⁺-ATPase inhibitor which fails to hit a solid particle or reaches a particle whose layer has already solidified (spray drying) so that the droplet does not stick on collision with the particle, can serve as a nuclei. In another embodiment, nuclei can be formed by interparticular collision, abrasion and destruction of particles. For example, dust produced following the collision of two solid particles serves as a nucleus for new particle growth.
*c*) Selecting out a microparticle that has a desired size distribution, e.g., of less than 250 µm, e.g., a size distribution of between 50-200 µm, 50-150 µm or 100-180 µm. The microparticles of a desired size are selected from the fluidized bed using any known method of selecting out a microparticle from a fluidized bed. In one example, the microparticle is selected out using a countercurrent gravity classifier. For example, the microparticles can be selected using a zigzag classifier. The classifier allows very precise control of the grain size by means of a classifying air stream. The micropellet entering the classifier, forced by gravity, moves downwards on the bottom wall of the classifying duct. At every bend of the classifying duct, the material must pass through the classifying air flow to reach the opposite wall. On its way, the micropellet moves essentially in vertical direction to the classifying air flow. Consequently, across-flow classification occurs at every bend of the duct. Much of the finer micropellet stream with slow floating speed is forced out of the granular stream and carried upwards. To make separation complete, the selection process occurs at several successive bends of the duct. Particles that are eliminated from the discharged material are carried upwards and depending on their size, enter the bed again at shorter or greater distance from the nozzle. Hence, the smaller and lighter particles enter the bed at greater distance from the nozzle. The larger particles are classified and sprayed more often until their size allows them to pass the classifier on the way down.

### Formulating and administering the microparticles

The microparticles described herein can be formulated into pharmaceutical compositions by admixing with pharmaceutically acceptable nontoxic excipients and carriers. Such compositions can be prepared for administration by various routes, but preferably the composition should be administered orally. The microparticles can be processed into solutions, suspensions, emulsions, gels, tablets, effervescent tablets, powder in sachets, coated tablets or filled into capsules.

Since H⁺,K⁺-ATPase inhibitors are susceptible to degradation/transformation in acidic and neutral media, the oral solid dosage form of microparticles must be protected from contact with the acidic gastric juice and the H⁺,K⁺-ATPase inhibitor. This can be done by coating the microparticles with an enteric coating. The small microparticles described herein have good mechanical strength and can withstand processing with a polymer coating in a fluid bed.

Particles of large diameter, e.g., particles having a diameter of greater than 400 µm, produce a roughness in the mouth when administered orally in a liquid dosage form. Since the microparticles of the invention are less than 250 µm, the sensation of roughness in the mouth is eliminated making them ideal for liquid and solid dosage formulations.

Examples of liquid dosage forms can include pharmaceutically acceptable emulsions, microemulsions, solutions, suspensions, syrups and elixirs. The liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils) glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, e.g., effervescent tablets, fast dissolving tablets\disintegrating, pills, powders, and granules. In such solid dosage forms, the microparticles described herein can be mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, 3) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and I) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In a particularly perferred embodiment, the microparticles described herein are processed into a multiple unit tablet which has fast dissolving\disintegrating properties in the oral cavity, or which can dissolve\disintegrate rapidly in water before being orally administered.

### Coasting

The microparticles described herein are preferably coated with an enteric coating. Methods of coating particles are known in the art. For example, before applying enteric coating layer(s) onto the microparticle, the microparticle may optionally be covered with one or more separating layers comprising pharmaceutical excipients optionally including alkaline compounds such as for instance pH-buffering compounds. This/these separating layer(s) separate(s) the microparticle from the outer layer(s) being enteric coating layer(s).

The separating layer(s) can be applied to the core material by coating or layering procedures using suitable equipment such as in a fluidized bed apparatus using water and/or organic solvents for the coating process. As an alternative the separating layer(s) can be applied to the core material by using powder coating technique. The materials for separating layers are pharmaceutically acceptable compounds such as, for instance, sugar, polyethylene glycol, polyvinylpyrrolidone, polyvinyl alcohol, polyvinyl acetate, hydroxypropyl cellulose, methyl-cellulose, ethylcellulose, hydroxypropyl methyl cellulose, carboxymethylcellulose sodium and others, used alone or in mixtures. Additives such as plasticizers, colorants, pigments, fillers, anti-tacking and anti-static agents, such as for instance magnesium stearate, titanium dioxide, talc and other additives may also be included into the separating layer(s). The optionally applied separating layer(s) is not essential for the invention. However the separating layer(s) may improve the chemical stability of H⁺,K⁺-ATPase inhibitor and/or the physical properties of the novel multiple unit tableted dosage form.

One or more enteric coating layers are applied onto the microparticle using a suitable coating technique known in the art. The enteric coating layer material may be dispersed or dissolved in either water or in suitable organic solvents. As enteric coating layer polymers one or more, separately or in combination, of the following can be used; e.g. solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s).

The enteric coating layers may optionally contain pharmaceutically acceptable plasticizers to obtain the desired mechanical properties, such as flexibility and hardness of the enteric coating layers. Such plasticizers are for instance, but not restricted to, triacetin, citric acid esters, phthalic acid esters, dibutyl sebacate, cetyl alcohol, polyethylene glycols, polysorbates or other plasticizers.

The amount of plasticizer is optimised for each enteric coating layer formula, in relation to selected enteric coating layer polymer(s), selected plasticizer(s) and the applied amount of said polymer(s), in such a way that the mechanical properties, i.e. flexibility and hardness of the enteric coating layer(s), for instance exemplified as Vickers hardness, are adjusted so that the acid resistance of the pellets covered with enteric coating layer(s) does not decrease significantly during the compression of pellets into tablets. The amount of plasticizer is usually above 10 % by weight of the enteric coating layer polymer(s), preferably 15 - 50 % and more preferably 20 - 50 %. Additives such as dispersants, colorants, pigments, polymers e.g. poly(ethylacrylat, methylmethacrylat), anti-tacking and anti-foaming agents may also be included into the enteric coating layer(s). Other compounds may be added to increase film thickness and to decrease diffusion of acidic gastric juices into the acidic susceptible material.

To protect the H⁺,K⁺-ATPase inhibitors and to obtain an acceptable acid resistance the enteric coating layer(s) constitutes a thickness of approximately at least 10 µm, preferably more than 20 µm. The maximum thickness of the applied enteric coating layer(s) is normally only limited by processing conditions.

### Over-coating layer

Microparticles covered with enteric coating layer(s) may further be covered with one or more over-coating layer(s). The over-coating layer(s) can be applied to the enteric coating layered pellets by coating or layering procedures in suitable equipments such as in a fluidized bed apparatus using water and/or organic solvents for the layering process. The materials for over-coating layers are pharmaceutically acceptable compounds such as, for instance sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures. Additives such as plasticizers, colorants, pigments, fillers, anti-tacking and anti-static agents, such as for instance magnesium stearate, titanium dioxide, talc and other additives may also be included into the over-coating layer(s). Said over-coating layer may further prevent potential agglomeration of enteric coating layered pellets, protect the enteric coating layer towards cracking during the compaction process and enhance the tableting process. The maximum thickness of the applied over-coating layer(s) is normally only limited by processing conditions.

The microparticles achieved can be coated with a polymer to achieve a time-controlled release, a site-controlled release or a pH-dependent release. Suitable polymers for coating can be, but are not limited to, the same type of polymers as listed above.

### Uses of the microparticles containing H⁺,K⁺-ATPase inhibitors

The pharmaceutical compositions containing H⁺,K⁺-ATPase inhibitors as desribed herein, are useful for inhibiting gastric acid secretion in mammals and man. In a more general sense, they may be used for prevention and treatment of gastric acid related diseases in mammals and man, including e.g. reflux esophagitis, gastritis, duodenitis, gastric ulcer and duodenal ulcer. Furthermore, they may be used for treatment of other gastrointestinal disorders where gastric acid inhibitory effect is desirable e.g. in patients on NSAID therapy, in patients with Non Ulcer Dyspepsia, in patients with symptomatic gastro-esophageal reflux disease, and in patients with gastrinomas. They may also be used in patients in intensive care situations, in patients with acute upper gastrointestinal bleeding, pre-and postoperatively to prevent acid aspiration of gastric acid and to prevent and treat stress ulceration. Further, they may be useful in the treatment of psoriasis as well as in the treatment of Helicobacter infections and diseases related to these.

The typical daily dose of the H⁺,K⁺-ATPase inhibitor microparticle composition varies and will depend on various factors such as the individual requirements of the patients, the mode of administration and the disease. In general, the daily dose will be in the range of 1-400 mg of the H⁺,K⁺-ATPase inhibitor.

### Working examples

The following examples illustrate different aspects of the invention without limiting the scope.

### Example 1: Esomeprazole Mg

### Preparation of particles

Microparticles were prepared in a continuous fluidized bed system (Glatt AGT 150, Weimar, Germany) from a suspension of esomeprazole magnesium (Mg) (see EP 9592608.8). The suspension was made by dissolving hydroxypropylmethylcellulose 6 cps (223 g) and polysorbate 80 (29 g) into water (6955 g) and by dispersing esomeprazole Mg trihydrate (1486 g) with a high-shear mixer (Silverson). Solid content of the suspension was 20%w/w. The particle size of the suspended esomeprazole Mg was further reduced by wet milling to a median particle size of 5 µm determined by laser diffractometry.

The suspension was sprayed into a Glatt AGT 150 fluidized bed with a speed of 20-30 g/min. The nozzle had an opening of 0.8 mm. The inlet air flow was approximately 80-100 m³/h, inlet air temperature varied 80-88°C, atomizing air pressure 4.8 bar, sifter air pressure 45 mbar and sifter air flow 1.1 m³/h. Median size of the uncoated particles was 140 µm, 90% smaller than 173 µm and 10 % smaller than 113 µm when determined by laser diffractometry. Estimated from scanning electron micrographs, pores on the surface of the particles were smaller than 5 µm.

### Coating of particles

These microparticles (100 g) were subcoated in a fluidized bed. The composition of subcoat dispersion was:

| | |
|---|---|
| Hydroxypropylcellulose | 35 g |
| Talc | 60 g |
| Magnesium stearate | 5 g |
| Water | 700 g |

Furthermore, these subcoated particles (100 g) were coated with enteric coating. The composition of enteric coating dispersion was:

| | |
|---|---|
| Eudragit L30D dispersion | 401 g |
| Triethylcitrate | 36 g |
| Glyceryl monostearate | 6 g |
| Polysorbate 80 | 0.6 g |
| Water | 235 g |

The drug content of enteric coated particles was 124 mg esomeprazole/g. The acid resistance of the enteric coated particles after 2 h in 0.1 M hydrochloride acid was 90%.

### Compression of EC-coated particles

### Composition of esomeprazole microparticle tablets

| Esomeprazole Mg EC-coated microparticles | 13.07 g |
|---|---|
| Avicel PH 102 SCG | 19.00 g |
| Sodium stearyl fumarate, Pruv | 0.07 g |

Sodium stearyl fumarate was sieved through a 0.5 mm sieve before blending with other components in a Turbula mixer for 10 min. Tablets were compressed with an instrumented Korsch PH106 using three pairs of 7x14 oblong punches. The target weight for the tablets was 400 mg. Drug content was 20.1 mg esomeprazole/tablet and the results were consistent with this: 20.08 mg/tablet. The force maxima of upper punch were 6.3-6.9 kN, which resulted in tablets with breaking force of 95 N, RSD 6 %. The mean weight of tablets was 397 mg. The acid resistance of tablets after 2 h in 0.1 M hydrochloride acid was 88.5 %. This does not differ significantly from the acid resistance of enteric coated microparticles before compression, which shows that the coated microparticles endure compression.

### Example 2: Omeprazole Mg

### Preparation of particles

Microparticles were prepared in a continuous fluidized bed system (Glatt AGT 150, Weimar, Germany) from a suspension of omeprazole Mg (EP 97921045.7). The suspension was done by dissolving hydroxypropylmethylcellulose 6 cps (225 g) and polysorbate 80 (30 g) into water (4246 g) and by dispersing the omeprazole Mg (1500 g) in the mixture. Solid content of the suspension was 29 % (in weight). The particle size of the suspended esomeprazole Mg was further reduced by wet milling.

The suspension was sprayed into a Glatt AGT 150 fluidized bed with a speed of 20-30 g/min. The nozzle had a opening of 0.8 mm. The inlet air flow was approximately 100-115 m³/h, inlet air temperature varied 82-85°C, atomizing air pressure 4.8 bar, sifter air pressure 45-62 mbar and sifter air flow 1.1-1.3 m³/h. Median size of the uncoated particles was 164 µm, 90% smaller than 206 µm and 10 % smaller than 126 µm when determined by laser diffractometry. Estimated from the scanning electron back-scattering graphs of the cross-section of particles, the inner structure of particles is dense and homogeneous. Estimated from the scanning electron micrographs of the surface of the particles, the pores are smaller than 5 µm.

### Coating of particles

These microparticles (100 g) were subcoated upto 102 w/w% in a fluidized bed. The composition of subcoat dispersion was:

| | |
|---|---|
| Hydroxypropylcellulose | 36 g |
| Talc | 61 g |
| Magnesium stearate | 4.9 g |
| Water | 715 g |

Agglomerates larger than 315 µm were removed by sieving. The subcoated particles (100 g) were coated with enteric coating. The composition of enteric coating dispersion was:

| | |
|---|---|
| Eudragit L30D dispersion | 497 g |
| Triethylcitrate | 45 g |
| Glyceryl monostearate | 7.6 g |
| Polysorbate 80 | 0.76 g |
| Water | 292 g |

The drug content of enteric coated particles was 115 mg omeprazole/g. The acid resistance of the enteric coated particles after 2 h in 0.1 M hydrochloride acid was 95 %.

### Compression of EC-coated particles

The enteric coated microparticles were mixed with microcrystalline cellulose for 10 min in a Turbula mixer (W.A. Bachofen, Switzerland). Sodium stearyl fumarate was then added through a sieve and the final mixture was blended for 2 min. The composition of the mixture is given below:

| | |
|---|---|
| Enteric coated particles | 30.00 % |
| Microcrystalline cellulose | 69.86 % |
| Sodium stearyl fumarate | 0.14 % |

An amount of 436 mg of the mixture, corresponding to an omeprazole content of 15.0 mg, was individually weighed for each tablet on an analytical balance and manually filled into the die of a single punch press (Korsch EK 0, Germany). Compaction was then performed in the single punch press equipped with 11.3 mm flat-faced punches at a maximum compaction force of 4.3 ±0.2 kN. The hardness of the tablets was approximately 40 N (Schleuniger, Switzerland).

The reduction of acid resistance of enteric coated pellets caused by compression was 1%.

### Example 3: Esomeprazole Mg

### Preparation of particles

Microparticles were prepared in a continuous fluidized bed system (Glatt AGT 150, Weimar, Germany) from two suspensions of esomeprazole Mg trihydrate. The suspensions were done by dissolving hydroxypropylmethylcellulose 6 cps (223 g and 225 g) and polysorbate 80 (29.3 g and 29.6 g) into water (6955 g and 7020 g) and by dispersing the esomeprazole Mg trihydrate (1486 g and 1500 g) with a high-shear mixer (Silverson). Solid content of the suspensions were 20 % w/w. The particle size of the suspended esomeprazole Mg was further reduced by wet milling.

The suspension was sprayed into a Glatt AGT 150 fluidized bed with a speed of 20-30 g/min. The nozzle had a opening of 0.8 mm. The inlet air flow was approximately 80-100 m³/h, inlet air temperature varied 82-85°C and 86-87°C, atomizing air pressure was 4.8 bar, sifter air pressure 43-46 mbar and sifter air flow was 1.1 m³/h. Mean values of measured median size of the uncoated particles was 137 µm, 90 % smaller than 170 µm and 10 % smaller than 109 µm when determined by laser diffractometry. Estimated from the scanning electron micrographs of the surface of the particles, the pores are smaller than 5 µm.

### Coating of particles

The microparticles obtained from the two suspensions were blended and the agglomerates larger than 315 µm were removed. Microparticles (100 g) were subcoated upto 104 w/w% in a fluidized bed. The composition of subcoat dispersion was:

| | |
|---|---|
| Hydroxypropylcellulose | 37 g |
| Talc | 63 g |
| Magnesium stearate | 5 g |
| Water | 730 g |

Subcoated particles (100 g) were coated with enteric coating. The composition of enteric coating dispersion was:

| | |
|---|---|
| Eudragit L30D dispersion | 505 g |
| Triethylcitrate | 45 g |
| Glyceryl monostearate | 7.7 g |
| Polysorbate 80 | 0.77 g |
| Water | 297 g |

The drug content of enteric coated particles was 117 mg esomeprazole/g. The acid resistance of enteric coated particles after 2 h in 0.1 M hydrochloride acid was 90 %.

### Compression of enteric coated particles

The enteric coated microparticles were mixed with microcrystalline cellulose for 10 min in a Turbula mixer (W.A. Bachofen, Switzerland). Sodium stearyl fumarate was then added through a sieve and the final mixture was blended for 2 min. The composition of the mixture is given below:

| | |
|---|---|
| Enteric coated particles | 30.00 % |
| Microcrystalline cellulose | 69.86 % |
| Sodium stearyl fumarate | 0.14 % |

An amount of 437 mg of the mixture, corresponding to an omeprazole content of 15.0 mg, was individually weighed for each tablet on an analytical balance and manually filled into the die of a single punch press (Korsch EK 0, Germany). Compaction was then performed in the single punch press equipped with 11.3 mm flat-faced punches at a maximum compaction force of 4.1 ±0.2 kN. The hardness of the tablets was approximately 40 N (Schleuniger, Switzerland).

The reduction of acid resistance of enteric coated pellets caused by compression was 1%.

## Claims

1. A method of preparing a homogeneous microparticle comprising an acid labile H⁺\K⁺- ATPase inhibitor, the method comprising:
providing a granulation liquid medium having a solid content and comprising:
(i) an acid labile H⁺\K⁺- ATPase inhibitor, an alkaline salt thereof, or one of its single enantiomers, or an alkaline salt thereof,
(ii) a polymer selected from the group consisting of a water soluble or water insoluble polymer, wherein the polymer is at least 5 % by weight based on the solid content, and
(iii) a liquid in which the polymer is soluble or dispersible;
spraying the liquid medium into a fluidized bed; and
selecting out a microparticle that has a size distribution of the microparticle is between 50 µm to 250 µm, thereby obtaining a dry, homogeneous microparticle, wherein at least 80 % by weight of the microparticle based on its dry weight content is the acid labile H⁺\K⁺- ATPase inhibitor, the alkaline salt thereof, or one of its single enantiomers, or the alkaline salt thereof.

2. A method according to claim 1, wherein the desired size distribution of the microparticle is between 50 µm and 150 µm.

3. A method according to claim 1 wherein the solid content is from 15 to 70 weight %.

4. A method according to claim 3 wherein the solid content is from 15 to 60 weight %.

5. A method according to claim 4 wherein the solid content is from 20 to 50 weight %.

6. A method according to claim 1 wherein the granulation liquid medium is a suspension.

7. A method according to claim 1 wherein the granulation liquid medium is a solution.

8. A method according to claim 1 wherein the granulation liquid medium is an emulsion.

9. A method according to any of the preceeding claims wherein the acid labile H⁺\K⁺-ATPase inhibitor, the alkaline salt thereof, or one of its single enantiomers, or the alkaline salt thereof, has a percentage weight of between 80 to 95, based on the weight of the dried microparticle.

10. A method according to any of the preceeding claims wherein the solid content of the medium is from 15 to 70 weight % and the weight of the acid labile H⁺\K⁺-ATPase inhibitor, the alkaline salt thereof, or one of its single enantiomers, or the alkaline salt thereof, is from 80 to 95 % of the weight of the dried microparticle.

11. A method according to any of the preceeding claims wherein the polymer is selected from the group consisting of a cellulose derivative, a polysaccharide, a natural polymer, a synthetic polymer, a surfactant and mixtures thereof.

12. A method according to any of the preceeding claims wherein the liquid in which the polymer is soluble is selected from the group consisting of water, tertiary butyl alcohol, cyclohexane, methylene chloride, methanol, ethanol and mixtures thereof.

13. A method according to any of the preceding claims wherein the acid labile H⁺,K⁺-ATPase inhibitor is selected from the group consisting of omeprazole, an alkaline salt thereof, esomeprazole, and an alkaline salt thereof.

14. A method according to any of the preceding claims wherein the method further comprises coating the selected microparticle with an enteric coating layer.

15. A microparticle prepared according to the method of any of claims 1-14.

16. A homogeneous microparticle comprising an acid labile H⁺,K⁺- ATPase inhibitor, wherein the microparticle comprises:
(i) at least 80 % by weight based on the dry content of the microparticle of an acid labile H⁺,K⁺- ATPase inhibitor, or an alkaline salt thereof, or one of its single enantiomers, or an alkaline salt thereof, and
(ii) at least 5 % by weight based on the solid content of a polymer, wherein the polymer is a water soluble or water insoluble polymer.

17. A microparticle according to claim 16, wherein the microparticle has a size distribution in the range from 50 to 250 µm.

18. A microparticle according to claim 16, wherein the selected microparticle has a size distribution in the range from 50 to 150 µm.

19. A microparticle according to claim 16 further comprising an enteric coating.

20. The microparticle according to claim 16 wherein the acid labile H⁺,K⁺- ATPase inhibitor is selected from the group consisting of omeprazole, an alkaline salt thereof, esomeprazole and an alkaline salt thereof.

21. A pharmaceutical composition comprising the microparticle of claim 16.

22. A use of a microparticle according to claim 17 for the preparation of a medicament for the prophylaxis or treatment of a gastric acid related disease.

23. A use of a microparticle according to claim 22, wherein the gastric acid related disease is reflux esophagitis, gastritis, duodenitis, gastric ulcer or duodenal ulcer.

## Patentansprüche

1. Verfahren zur Herstellung eines homogenen Mikroteilchens, das einen säurelabilen H⁺\K⁺-ATPase-Inhibitor enthält, bei dem man:
ein Granulierflüssigkeitsmedium, das
(i) einen säurelabilen H⁺\K⁺-ATPase-Inhibitor oder ein alkalisches Salz davon oder eines seiner Einzelenantiomere oder ein alkalisches Salz davon,
(ii) ein Polymer aus der Gruppe bestehend aus einem wasserlöslichen oder wasserunlöslichen Polymer, wobei das Polymer mindestens' 5 Gew.-%, bezogen auf den Feststoffgehalt, ausmacht, und
(iii) eine Flüssigkeit, in der das Polymer löslich ist,
enthält, bereitstellt;
das Flüssigkeitsmedium in eine Wirbelschicht einsprüht und
ein Mikroteilchen mit einer Größenverteilung zwischen 50 µm und 250 µm ausselektiert, wodurch man ein trockenes, homogenes Mikroteilchen erhält, wobei es sich bei mindestens 80 Gew.-% des Mikroteilchens, bezogen auf das Trockengewicht des Mikroteilchens, um den säurelabilen H⁺\K⁺-ATPase-Inhibitor oder das alkalische Salz davon oder eines seiner Einzelenantiomere oder das alkalische Salz davon handelt.

2. Verfahren nach Anspruch 1, bei dem die gewünschte Größenverteilung des Mikroteilchens zwischen 50 µm und 150 µm liegt.

3. Verfahren nach Anspruch 1, bei dem der Feststoffgehalt 15 bis 70 Gew.-% beträgt.

4. Verfahren nach Anspruch 3, bei dem der Feststoffgehalt 15 bis 60 Gew.-% beträgt.

5. Verfahren nach Anspruch 4, bei dem der Feststoffgehalt 20 bis 50 Gew.-% beträgt.

6. Verfahren nach Anspruch 1, bei dem es sich bei dem Granulierflüssigkeitsmedium um eine Suspension handelt.

7. Verfahren nach Anspruch 1, bei dem es sich bei dem Granulierflüssigkeitsmedium um eine Lösung handelt.

8. Verfahren nach Anspruch 1, bei dem es sich bei dem Granulierflüssigkeitsmedium um eine Emulsion handelt.

9. Verfahren nach einem der vorhergehenden Ansprüche, bei dem der säurelabile H⁺\K⁺-ATPase-Inhibitor oder das alkalische Salz davon oder eines seiner Einzelenantiomere oder das alkalische Salz davon einen Gewichtsprozentanteil zwischen 80 und 95, bezogen auf das Gewicht des getrockneten Mikroteilchens, hat.

10. Verfahren nach einem der vorhergehenden Ansprüche, bei dem der Feststoffgehalt des Mediums 15 bis 70 Gew.-% beträgt und das Gewicht des säurelabilen H⁺\K⁺-ATPase-Inhibitors oder des alkalischen Salzes davon oder eines seiner Einzelenantiomere oder des alkalischen Salzes davon 80 bis 95% des Gewichts des getrockneten Mikroteilchens ausmacht.

11. Verfahren nach einem der vorhergehenden Ansprüche, bei dem man das Polymer aus der Gruppe bestehend aus einem Cellulosederivat, einem Polysaccharid, einem natürlichen Polymer, einem synthetischen Polymer, einem Tensid und Mischungen davon auswählt.

12. Verfahren nach einem der vorhergehenden Ansprüche, bei dem man die Flüssigkeit, in der das Polymer löslich ist, aus der Gruppe bestehend aus Wasser, tert.-Butylalkohol, Cyclohexan, Methylenchlorid, Methanol, Ethanol und Mischungen davon auswählt.

13. Verfahren nach einem der vorhergehenden Ansprüche, bei dem man den säurelabilen H⁺\K⁺-ATPase-Inhibitor aus der Gruppe bestehend aus Omeprazol, einem alkalischen Salz davon, Esomeprazol und einem alkalischen Salz davon auswählt.

14. Verfahren nach einem der vorhergehenden Ansprüche, bei dem man ferner das selektierte Mikroteilchen mit einer magensaftresistenten Schicht beschichtet.

15. Nach dem Verfahren gemäß einem der Ansprüche 1-14 hergestelltes Mikroteilchen.

16. Homogenes Mikroteilchen, das einen säurelabilen H⁺\K⁺-ATPase-Inhibitor enthält, wobei das Mikroteilchen:
(i) mindestens 80 Gew.-%, bezogen auf den Trockengehalt des Mikroteilchens, eines säurelabilen H⁺\K⁺-ATPase-Inhibitors oder eines alkalischen Salzes davon oder eines seiner Einzelenantiomere oder eines alkalischen Salzes davon und
(ii) mindestens 5 Gew.-%, bezogen auf den Feststoffgehalt, eines Polymers, wobei es sich bei dem Polymer um ein wasserlösliches oder wasserunlösliches Polymer handelt;
enthält.

17. Mikroteilchen nach Anspruch 16 mit einer Größenverteilung im Bereich von 50 bis 250 µm.

18. Mikroteilchen nach Anspruch 16 mit einer Größenverteilung im Bereich von 50 bis 150 µm.

19. Mikroteilchen nach Anspruch 16, das ferner einen magensaftresistenten Überzug aufweist.

20. Mikroteilchen nach Anspruch 16, bei dem der säurelabile H⁺\K⁺-ATPase-Inhibitor aus der Gruppe bestehend aus Omeprazol, einem alkalischen Salz davon, Esomeprazol und einem alkalischen Salz davon ausgewählt ist.

21. Pharmazeutische Zusammensetzung, enthaltend das Mikroteilchen gemäß Anspruch 16.

22. Verwendung eines Mikroteilchens gemäß Anspruch 17 zur Herstellung eines Arzneimittels für die Prophylaxe oder Behandlung einer mit Magensäure in Zusammenhang stehenden Erkrankung.

23. Verwendung eines Mikroteilchens nach Anspruch 22, bei der es sich bei der mit Magensäure in Zusammenhang stehenden Erkrankung um Reflux-ösophagitis, Gastritis, Duodenitis, Magengeschwüre oder Zwölffingerdarmgeschwüre handelt.

## Revendications

1. Méthode de préparation d'une microparticule homogène comprenant un inhibiteur d'H⁺/K⁺-ATPase labile à l'acide, la méthode comprenant :
la fourniture d'un milieu liquide de granulation ayant une teneur en matière sèche et comprenant :
(i) un inhibiteur d'H⁺/K⁺-ATPase labile à l'acide, un sel alcalin de celui-ci, ou l'un de ses énantiomères uniques, ou un sel alcalin de celui-ci,
(ii) un polymère choisi parmi le groupe constitué par un polymère soluble dans l'eau ou insoluble dans l'eau, où le polymère constitue au moins 5% en poids sur la base de la teneur en matière sèche, et
(iii) un liquide dans lequel le polymère est soluble ou dispersable ;
la pulvérisation du milieu liquide dans un lit fluidisé ; et
la sélection d'une microparticule ayant une granulométrie de microparticule allant de 50 µm à 250 µm, obtenant ainsi une microparticule homogène sèche, où au moins 80% en poids de la microparticule sur la base de sa teneur en matière sèche est l'inhibiteur d'^{H}+/^{K}+-ATPase labile à l'acide, un sel alcalin de celui-ci, ou l'un de ses énantiomères uniques, ou un sel alcalin de celui-ci.

2. Méthode selon la revendication 1, dans laquelle la granulométrie désirée de la microparticule est comprise entre 50 µm et 150 µm.

3. Méthode selon la revendication 1, dans laquelle la teneur en matière sèche va de 15 à 70% en poids.

4. Méthode selon la revendication 3, dans laquelle la teneur en matière sèche va de 15 à 60% en poids.

5. Méthode selon la revendication 4, dans laquelle la teneur en matière sèche va de 20 à 50% en poids.

6. Méthode selon la revendication 1, dans laquelle le milieu liquide de granulation est une suspension.

7. Méthode selon la revendication 1, dans laquelle le milieu liquide de granulation est une solution.

8. Méthode selon la revendication 1, dans laquelle le milieu liquide de granulation est une émulsion.

9. Méthode selon l'une quelconque des revendications précédentes, dans laquelle l'inhibiteur d'H⁺/K⁺-ATPase labile à l'acide, un sel alcalin de celui-ci, ou l'un de ses énantiomères uniques, ou un sel alcalin de celui-ci, a un pourcentage pondéral allant de 80 à 95, sur la base du poids de la microparticule séchée.

10. Méthode selon l'une quelconque des revendications précédentes, dans laquelle la teneur en matière sèche du milieu va de 15 à 70% en poids, et le poids de l'inhibiteur d'H⁺/K⁺-ATPase labile à l'acide, d'un sel alcalin de celui-ci, ou de l'un de ses énantiomères uniques, ou d'un sel alcalin de celui-ci, va de 80 à 95% du poids de la microparticule séchée.

11. Méthode selon l'une quelconque des revendications précédentes, dans laquelle le polymère est choisi parmi le groupe constitué par un dérivé de cellulose, un polysaccharide, un polymère naturel, un polymère synthétique, un agent tensioactif et des mélanges de ceux-ci.

12. Méthode selon l'une quelconque des revendications précédentes, dans laquelle le liquide dans lequel le polymère est soluble est choisi parmi le groupe constitué par l'eau, l'alcool tertio-butylique, le cyclohexane, le chlorure de méthylène, le méthanol, l'éthanol et des mélanges de ceux-ci.

13. Méthode selon l'une quelconque des revendications précédentes, dans laquelle l'inhibiteur d'H⁺/K⁺-ATPase labile à l'acide est choisi parmi le groupe constitué par l'oméprazole, un sel alcalin de celui-ci, l'ésoméprazole, et un sel alcalin de celui-ci.

14. Méthode selon l'une quelconque des revendications précédentes, dans laquelle la méthode comprend en outre l'enrobage de la microparticule sélectionnée par une couche d'enrobage gastro-résistant.

15. Microparticule préparée selon la méthode selon l'une quelconque des revendications 1-14.

16. Microparticule homogène comprenant un inhibiteur d'H⁺/K⁺-ATPase labile à l'acide, dans laquelle la microparticule comprend :
(i) au moins 80% en poids de la teneur en matière sèche de la microparticule d'un inhibiteur d'H⁺/K⁺-ATPase labile à l'acide, ou d'un sel alcalin de celui-ci, ou de l'un de ses énantiomères uniques, ou d'un sel alcalin de celui-ci, et
(ii) au moins 5% en poids de la teneur en matière sèche d'un polymère, où le polymère est un polymère soluble dans l'eau ou insoluble dans l'eau.

17. Microparticule selon la revendication 16, dans laquelle la microparticule a une granulométrie dans la plage allant de 50 à 250 µm.

18. Microparticule selon la revendication 16, dans laquelle la microparticule sélectionnée a une granulométrie dans la plage allant de 50 à 150 µm.

19. Microparticule selon la revendication 16, comprenant en outre un enrobage gastro-résistant.

20. Microparticule selon la revendication 16, dans laquelle l'inhibiteur d'H⁺/K⁺-ATPase labile à l'acide est choisi parmi le groupe constitué par l'oméprazole, un sel alcalin de celui-ci, l'ésoméprazole, et un sel alcalin de celui-ci.

21. Composition pharmaceutique comprenant la microparticule selon la revendication 16.

22. Utilisation d'une microparticule selon la revendication 17, pour la préparation d'un médicament destiné à la prophylaxie ou au traitement d'une maladie liée à l'acide gastrique.

23. Utilisation d'une microparticule selon la revendication 22, dans laquelle la maladie liée à l'acide gastrique est l'oesophagite peptique, la gastrite, la duodénite, l'ulcère gastrique ou l'ulcère duodénal.
